# EUROPEAN PATENT APPLICATION

(11) **EP 4 163 397 A1**
(43) Date of publication of application: **12.04.2023**
(21) Application number: 21816989.4
(22) Date of filing: 04.06.2021
(51) Int. Cl.: C12Q 1/70, A61B 10/00, A61B 5/08

(54) **SPECIMEN TRANSPORT KIT FOR DETECTING RESPIRATORY PATHOGENS AND METHODS FOR DETECTING RESPIRATORY PATHOGENS USING SAME**

(30) Priority: 05.06.2020 KR 20200068658
(71) Applicant: Seegene, Inc., Seoul 05548 (KR); SG Medical Inc., Songpa-gu Seoul 05548 (KR)
(72) Inventor: CHUN, Jong Yoon, Seoul 06075 (KR); LEE, Ji-Chul, Gwangmyeong-si Gyeonggi-do 14244 (KR); KWON, Hyuek-June, Seoul 07545 (KR); KO, Young-Jin, Hanam-si Gyeonggi-do 12966 (KR); LEE, Sangyeop, Hanam-si Gyeonggi-do 12904 (KR)
(74) Representative: Engelhard, Markus
(86) International application number: PCT/KR2021/007011
(87) International publication number: WO 2021/246820

(57) **Abstract**

The present invention relates to a specimen transport kit for detecting respiratory pathogens, which is configured to collect both a nasopharyngeal cavity specimen and a salivary specimen in a swab method and make a specimen transport medium impregnated with the specimens together, and a method for detecting respiratory pathogens using same. The present invention provides a higher positive rate and more accurate diagnostic results for respiratory pathogens than when the nasopharyngeal cavity specimen or the salivary specimen is used separately.

## Description

### Technical Field

### Cross-Reference to Related Application

This application claims priority from Korean Patent Application No. 2020-0068658, filed on 5 June 2020 in the Korean Intellectual Property Office, the disclosure of which is incorporated herein by reference.

### Technical Field

The present invention relates to sample transport kits for detecting respiratory pathogens and methods for detecting respiratory pathogens by using the same.

### Background Art

Respiratory diseases cause serious mortality, especially in children, the elderly, and immunocompromised people. Identifying causative pathogens is very important for infection control and appropriate patient care. Respiratory pathogen testing has developed rapidly with the development of molecular diagnostic technology, especially real-time PCR. Real-time PCR is very sensitive and prompt, and can simultaneously detect various pathogens compared with conventional methods.

Various upper respiratory tract (URT) samples and lower respiratory tract samples have been used for respiratory pathogen testing by molecular diagnosis.

In general, sample types such as throat swab samples and nasal swab samples as upper respiratory tract samples are widely used due to relatively convenient collection and low invasiveness.

Sputum and bronchoalveolar lavage (BAL) samples are used as lower respiratory tract samples.

In recent years, real-time PCR diagnostic kits are greatly spotlighted due to the 2019-nCov (novel coronavirus) pandemic. For coronavirus diagnosis, nasopharyngeal swab samples and oropharyngeal swab samples are used as upper respiratory tract samples, and sputum and bronchoalveolar lavage are used as lower respiratory tract samples. The nasopharyngeal and oropharyngeal swab samples are obtained by collecting secretions from the nasopharynx and oropharynx using cotton swabs and then stored in transport media. The sputum is collected and stored in a sterile vessel after the mouth is rinsed with saline.

However, lower respiratory tract samples are difficult to collect and may require pretreatment before nucleic acid extraction, and especially, sputum samples cannot be applied in the early stages of infection in which patients have substantially no cough symptoms or have a dry cough.

As an alternative to these upper and lower respiratory tract samples, a throat wash sample or a saliva sample has been suggested (Wang et al., Emerging Infectious Diseases, www.cdc.gov/eid, Vol. 10, No. 7, July 2004). However, the validity for the determination of respiratory infection has not yet been established in a throat wash sample or a saliva sample, and a sampling kit for effectively collecting, preserving, and transporting a throat wash sample or a saliva sample has not been disclosed.

Under the above-described circumstances in the art, there is still a need for effective and convenient sampling for detecting respiratory pathogens.

Throughout this application, various patents and publications are referenced and citations are provided in parentheses. The disclosure of these patents and publications in their entities are hereby incorporated by references into this application in order to more fully describe this invention and the state of the art to which this invention pertains.

### Disclosure of Invention

### Technical Problem

The present inventors have conducted intensive research to develop a novel sampling method capable of detecting respiratory pathogens. As a result, the present inventors established that improved detection rates of positives and more accurate diagnostic results for respiratory pathogens can be obtained when a nasopharyngeal sample and a saliva sample, collected by swabbing, are dipped together in a sample transport medium and then nucleic acid molecules released from the swabs are used, compared with when the nasopharyngeal sample or the saliva sample is used alone, and thus completed the present invention.

Accordingly, it is an object of the present invention to provide a method for detecting respiratory pathogens.

It is another object of the present invention to provide a sample transport kit for detecting respiratory pathogens.

Other objects and advantages of the present invention will become apparent from the detailed description to follow taken in conjugation with the appended claims and drawings.

### Solution to Problem

In an aspect of the present invention, there is provided a method for detecting a respiratory pathogen, the method comprising:
(a) dipping a nasopharyngeal swab and a saliva swab with collected samples into a sample transport medium contained in a tube;
(b) performing a nucleic acid amplification reaction using nucleic acid molecules obtained from the samples released from the nasopharyngeal swab and saliva swab, and a nucleic acid amplification reaction composition; and
(c) analyzing the results of the nucleic acid amplification reaction to determine the presence or absence of the respiratory pathogen.

The present inventors have made intensive researches to develop a novel sampling method capable of detecting respiratory pathogens. As a result, the present inventors established that improved detection rates of positives and more accurate diagnostic results for respiratory pathogens can be obtained when a nasopharyngeal sample and a saliva sample, collected by swabbing, are dipped together in a sample transport medium and then nucleic acid molecules released from the swabs are used, compared with when the nasopharyngeal sample or the saliva sample is used alone.

The detection method of the present invention will be described in detail according to each step as follows:

### Step (a): Dipping a nasopharyngeal swab and a saliva swab with collected samples into a sample transport medium

A nasopharyngeal swab and a saliva swab with collected samples are dipped into a sample transport medium contained in a tube.

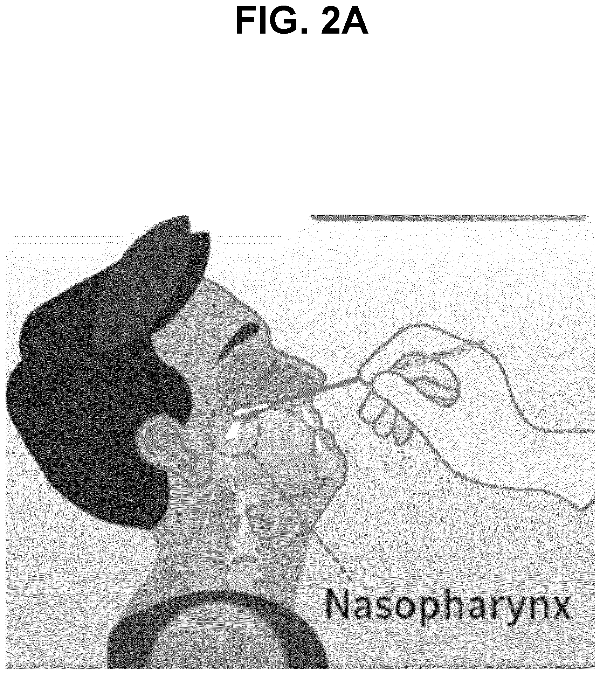

The sample collection using a nasopharyngeal swab may be performed by a general method known in the art. For example, referring to FIG. 2A, a nasopharyngeal swab is pushed, passing through the nostril, at an angle parallel to the roof of the mouth, and secretions under the middle of the inferior nasal concha are scraped to be collected, and then the nasopharyngeal swab is left under the middle of the inferior nasal concha for a few seconds such that the secretions can be absorbed into a fibrous layer of the swab.

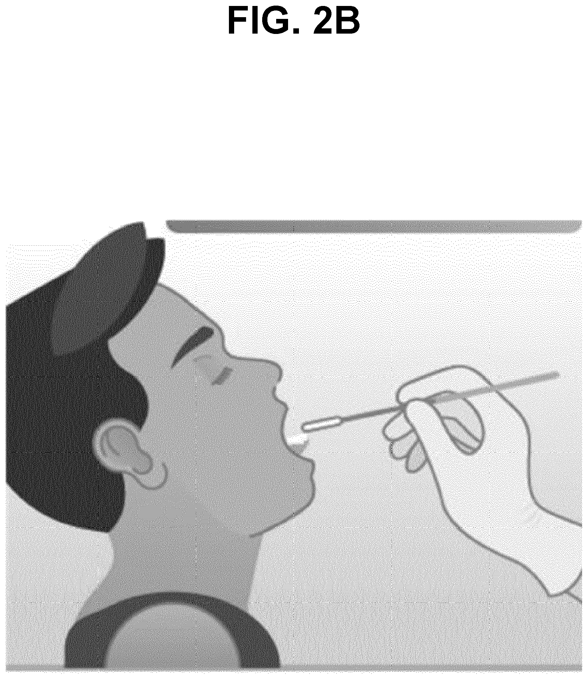

The sample collection using a saliva swab may be performed by allowing saliva to be sufficiently absorbed into the swab while swabbing the bottom portion of the tongue, or by collecting saliva in the end of the tongue and then allowing the saliva to be sufficiently absorbed into a head part of a fibrous layer of the saliva swab (see FIG. 2B).

The entire shape of the swab used in the present invention may be known in the art (see U.S. Patent No. 10327741). For example, the swab may have a support part and a fibrous layer part formed on the end of the support part.

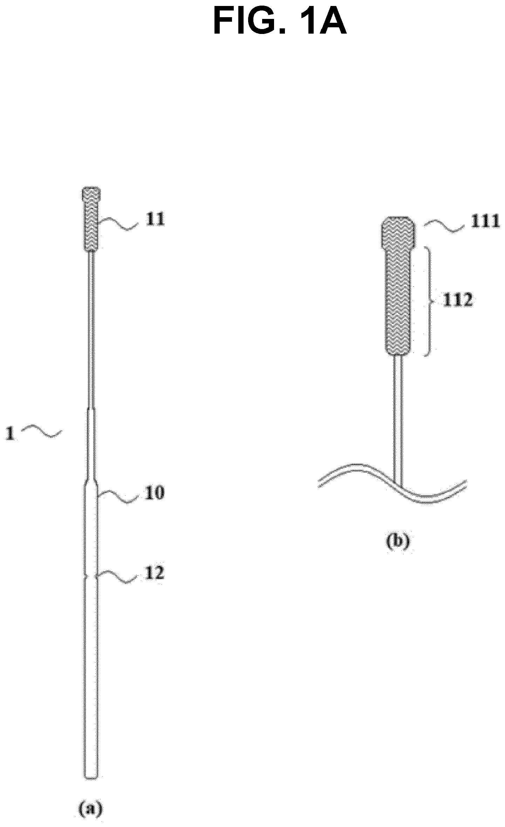

FIG. 1A illustrates an example of the nasopharyngeal swab usable in the present invention. A nasopharyngeal swab **1** includes a support part **10** and a fibrous layer part **11.** The fibrous layer part **11** includes a head part fibrous layer **111** and a pillar part fibrous layer **112.** The nasopharyngeal swab **1** includes a cutting point **12.**

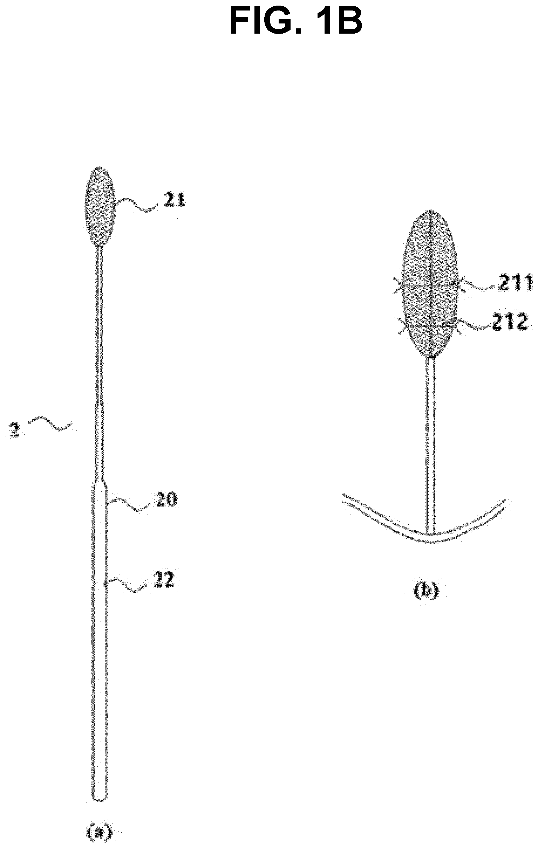

FIG. 1B illustrates an example of the saliva swab usable in the present invention. A saliva swab **2** includes a support part **20** and a fibrous layer part **21.** The fibrous layer part **21** has an elliptical shape with no division of a head part and a pillar part. The saliva swab **2** include a cutting point **22.**

Herein, the thickness of a fibrous layer refers to the length of the axis perpendicular to the longitudinal axis of the swab in the fibrous layer.

The fibrous layer at the end of the nasopharyngeal swab has a thickness of 3-4.0 mm. The fibrous layer at the end of the saliva swab has a thickness of 2.5-5.5 mm.

More specifically, the fibrous layer at the end of the nasopharyngeal swab may include a head part fibrous layer and a pillar part fibrous layer, and the head part fibrous layer may have a fibrous layer with a thickness of 3.7-3.9 mm and the pillar part fibrous layer may have a fibrous layer with a thickness of 3.0-3.1 mm.

More specifically, the fibrous layer of the saliva swab has an elliptical shape. The fibrous layer with an elliptical shape includes a horizontal long axis portion **211** and a horizontal short axis portion **212.**

As used herein while reciting the fibrous layer of the saliva swab, the term "horizontal long axis portion" refers to a portion representing the longest axial length among axes perpendicular to the longitudinal axis of the length direction of the swab in the fibrous layer having an elliptical shape, and the term "horizontal short axis portion" refers to an axis portion other than the portion representing the longest axial length among the axes perpendicular to the longitudinal axis.

Since the fibrous layer of the saliva swab has an elliptical shape, the axis representing the longest axial length in the fibrous layer represents the long axis, and the axis representing the shortest axial length represents the short axis, and both ends of the long axis and the short axis indicate vertices.

The thickness of the fibrous layer is largest at the horizontal long axis portion **211,** and as the fibrous layer approaches the vertices of the long axis, the thickness of the fibrous layer of horizontal short axis portion **212** decreases, and the thickness of the fibrous layer of horizontal short axis portion **212** is the smallest at the vertices of the long axis. Specifically, the thickness of the fibrous layer is 4.5-5.5 mm at the horizontal long axis portion **211** and 2.5-3.5 mm at the horizontal short axis portion **212.** More specifically, the thickness of the fibrous layer is 4.8-5.2 mm at the horizontal long axis portion **211** and 2.8-3.2 mm at the horizontal short axis portion **212.**

Fibers used in the formation of the fibrous layer may have a Dtex value of 3.00-3.50 and more specifically, a Dtex value of 3.30-3.35.

The above-described thickness and Dtex value of the fibrous layer allow the nasopharyngeal and saliva swabs to have appropriate absorbing ability and, when dipped in a sample transport medium, to have appropriate releasing ability.

The fibers of the fibrous layer are a substance having hydrophilic properties, and examples thereof include polyesters, polyamides, and cotton.

The fibrous layer may be formed by various methods known in the art, and for example, the fibrous layer may be formed by a flocking method.

The nasopharyngeal swab and saliva swab with collected samples are dipped, that is, impregnated in a sample transport medium contained in a tube. The sample contained in the swabs is released to the sample transport medium by such dipping. In general, the tube containing the sample transport medium in which the swabs are dipped is usually transported to a site where a nucleic acid amplification reaction is performed.

The sample transport medium usable in the present invention includes two types of media: a maintaining buffer and a lysis buffer (*i.e.,* an inactivating buffer). The maintaining buffer refers to a buffer that maintains a sample not to be lysed. The inactivating buffer lyses a sample, for example, viruses to allow a nucleic acid molecule in the viruses to be released into the buffer, denatures protein components to inactivate DNase and RNase, and maintains the integrity of the nucleic acid molecule.

More specifically, the sample transport medium used in the present invention is a lysis buffer.

According to a specific embodiment of the present invention, the sample transport medium contains (i) a chaotropic agent, (ii) a detergent, (iii) a chelator, and (iv) a buffer. Optionally, the sample transport medium may further contain a reducing agent.

Specifically, the chaotropic agent is guanidine thiocyanate, guanidine isocyanate, guanidine hydrochloride, and potassium thiocyanate, and more specifically, guanidine thiocyanate. The chaotropic agent opens microbial cells to induce cell lysis and allow the release of DNA and RNA, and inhibits the degradation of nucleic acid molecules by nucleases.

Specifically, the detergent is sodium dodecyl sulfate, lithium dodecyl sulfate, sodium taurodeoxycholate, sodium taurocholate, sodium glycocholate, sodium deoxycholate, sodium cholate, sodium alkylbenzene sulfonate, polysorbate, Triton X-100, or N-lauroyl sarcosine.

Specifically, the chelator is ethylene glycol tetraacetic acid, hydroxyethylethylenediaminetriacetic acid, diethylene triamine pentaacetic acid, N,N-bis(carboxymethyl)glycine, ethylenediaminetetraacetic acid, citrate anhydrous, sodium citrate, calcium citrate, ammonium citrate, ammonium bicitrate, citric acid, diammonium citrate, ferric ammonium citrate, or lithium citrate.

Specifically, the buffer is tris(hydroxymethyl)aminomethane, citrate, 2-(N-morpholino)ethanesulfonic acid, N,N-bis(2-hydroxyethyl)-2-aminoethanesulfonic acid, 1,3-bis(tris(hydroxymethyl)methyl amino)propane, 4-(2-hydroxyethyl)-1-piperazine ethanesulfonic acid, 3-(N-morpholino)propanesulfonic acid, hydroxyethyl piperazine ethane sulfonic acid, bicarbonates, and phosphates.

Specifically, the reducing agent is 2-mercaptoethanol, tris(2-carboxyethyl)phosphine, dithiothreitol, dimethylsulfoxide, and sodium hydroxide.

In the sample transport medium used in the present invention, the chaotropic agent may be contained in an amount of 5-30 parts by weight relative to 100 parts by weight of the medium, and the detergent, the chelator, the buffer, and the reducing agent may be contained in amounts of 1-15 parts by weight, 0.1-5 parts by weight, 2-10 parts by weight, and 0.1-3 parts by weight, respectively.

According to an embodiment, the sample transport medium performs an inactivation function by lysis of a respiratory infection pathogen and a stabilization function for a nucleic acid material (specifically, DNA or RNA, and more specifically RNA) released from the lysed pathogen.

Optionally, the sample transport medium further contains a pH indicator (*e.g.*, phenol red, bromocresol purple, and bromothymol blue) and, more specifically, phenol red. The pH indicator makes it possible to monitor whether the sample transport medium is infected or not with bacteria.

### Step (b): Performing nucleic acid amplification reaction

Then, a nucleic acid amplification reaction is performed using nucleic acid molecules obtained from the samples released from the nasopharyngeal swab and saliva swab, and a nucleic acid amplification reaction composition.

The obtaining of the nucleic acid molecules from the samples may be performed by various methods known in the art. According to an embodiment of the present invention, the nucleic acid molecules are isolated from the same by a magnetic particle-based isolation method (see U.S. Patent Nos. 6027945 and 7517969).

For example, the nucleic acid molecules can be isolated by sequentially using buffers for nucleic acid molecule isolation, including a lysis buffer, a binding buffer, a washing buffer, and a release buffer. More specifically, the samples are treated with a lysis buffer containing a chaotropic agent (*e.g.,* guanidine thiocyanate) and a detergent (*e.g.*, Tween-20) to allow viruses or bacteria in the sample to be lysed, and the resultant product of lysis is treated with a binding buffer containing magnetic particles to allow the nucleic acid molecule to be bound to the surface of the magnetic particles, and then the magnetic particles are washed with a washing buffer containing ethanol, and then treated with an eluting buffer, thereby separating the nucleic acid molecule bound to the surface of the magnetic particles.

Thereafter, the nucleic acid amplification reaction is performed. In the nucleic acid amplification reaction, probes and primers hybridizing with targets may be used.

The probe or primer used in the present invention has a sequence complementary to the target nucleotide sequence. As used herein, the term "complementary" refers to having complementarity to be selectively hybridizable with the above-described nucleotide sequence under specific hybridization or annealing conditions. Therefore, the term "complementary" has a different meaning from the term "perfectly complementary", and the primers or probe of the present invention may have one or more mismatch nucleotide sequences as long as the primers or probe is selectively hybridizable with the above nucleotide sequence.

As used herein, the term "primer" refers to a single-strand oligonucleotide capable of acting as an initial point of template-directed DNA synthesis at an appropriate temperature in an appropriate buffer under appropriate conditions (*i.e.*, four types of different nucleoside triphosphates and polymerase). The appropriate length of the primer may vary according to various factors, for example, temperature and use of the primer, but the primer is typically 15 to 30 nucleotides in length. A short primer molecule generally requires a lower temperature in order to form a sufficiently stable hybrid complex together with a template.

The primer sequence is not required to be perfectly complementary to a partial sequence of the template, and it is enough that the primer sequence has sufficient complementarity within a range in which the primer can make an intrinsic action when hybridized with the template. Therefore, the primer of the present invention does not need to have a perfectly complementary sequence to the above-described nucleotide sequence, which is the template, and it is enough that the primer has a sufficient complementarity within a range in which the primer can perform a primer action when hybridized with the gene sequence. Such a primer can be easily designed with reference to the above-described nucleotide sequence by a person skilled in the art, and for example, the design can be carried out using a computer program for primer design (*e.g.*, PRIMER 3 program).

As used herein, the term "probe" refers to a linear oligomer of natural or modified monomers or linkages, and the probe includes deoxyribonucleotides and ribonucleotides, can specifically hybridize with a target nucleotide sequence, and is naturally present or artificially synthesized. The probe of the present invention is preferably in a single-stranded form, and is an oligodeoxyribonucleotide.

As used herein, the term "amplification" refers to a reaction for amplifying nucleic acid molecules. A variety of amplification reactions have been reported in the art, including polymerase chain reaction (PCR, U.S. Patent Nos. 4,683,195, 4,683,202 and 4,800,159), reverse transcription-polymerase chain reaction (RT-PCR, Sambrook et. al., Molecular Cloning. A Laboratory Manual, 3rd ed. Cold Spring Harbor Press (2001)), methods by Miller, H. I. (WO 89/06700) and Davey, C. et al. (EP 329,822), ligase chain reaction (LCR), Gap-LCR (WO 90/01069), repair chain reaction (EP 439,182), transcription-mediated amplification (TMA, WO 88/10315), self-sustained sequence replication (WO 90/06995), selective amplification of target polynucleotide sequences (U.S. Patent No. 6,410,276), consensus sequence primed polymerase chain reaction (CP-PCR, U.S. Patent No. 4,437,975), arbitrarily primed polymerase chain reaction (AP-PCR, U.S. Patent Nos. 5,413,909 and 5,861,245), nucleic acid sequence based amplification (NASBA, U.S. Patent Nos. 5,130,238, 5,409,818, 5,554,517, and 6,063,603), strand displacement amplification, and loop-mediated isothermal amplification (LAMP), but is not limited thereto. Other usable amplification methods are disclosed in U.S. Patent Nos. 5,242,794, 5,494,810, and 4,988,617, and U.S. Patent Application Serial No. 09/854,317.

PCR is the most well-known method for nucleic acid amplification, and modifications and applications thereof have been developed. For example, for improving PCR specificity or sensitivity, touchdown PCR, hot start PCR, nested PCR, and booster PCR have been developed by modifying the conventional PCR procedure. In addition, real-time PCR, differential display PCR (DD-PCR), rapid amplification of cDNA ends (RACE), multiplex PCR, inverse polymerase chain reaction (IPCR), Vectorette PCR, and thermal asymmetric interlaced PCR (TAIL-PCR) have been developed for specific applications. The details of PCR are disclosed in McPherson, M. J., and Moller, S.G. PCR. BIOS Scientific Publishers, Springer-Verlag New York Berlin Heidelberg, N.Y. (2000), the teachings of which are incorporated herein by reference.

Most of respiratory pathogens have RNA as genome. In such a case, a gene amplification reaction is performed using primers to be hybridized with RNA or cDNA while RNA in a sample is used as a template. cDNA is synthesized from isolated RNA, and this cDNA is amplified. cDNA can be easily synthesized using reverse transcriptase (see PNAS USA, 85:8998(1988); Libert F, et al., Science, 244:569(1989); and Sambrook, J. et al., Molecular Cloning. A Laboratory Manual, 3rd ed. Cold Spring Harbor Press (2001)). Then, the synthesized cDNA is amplified through a gene amplification reaction.

The primers used for the present invention are hybridized or annealed to a region of the template to form a double-chain structure. Nucleic acid hybridization conditions suitable for forming such a double-chain structure are disclosed in Joseph Sambrook, et al., Molecular Cloning, A Laboratory Manual, Cold Spring Harbor Laboratory Press, Cold Spring Harbor, N. Y. (2001), and Haymes, B. D., et al., Nucleic Acid Hybridization, A Practical Approach, IRL Press, Washington, D.C. (1985).

A variety of DNA polymerases may be used in the amplification of the present invention, and include "Klenow" fragment of E. coli DNA polymerase I, a thermostable DNA polymerase, and bacteriophage T7 DNA polymerase. Preferably, the polymerase is a thermostable DNA polymerase obtained from a variety of bacterial species, including Thermus aquaticus (Taq), Thermus thermophilus (Tth), Thermus filiformis, Thermis flavus, Thermococcus literalis, and Pyrococcus furiosus (Pfu).

When a polymerization reaction is performed, the ingredients necessary for the reaction are preferably provided in excess to a reaction container. The excess with respect to the ingredients necessary for the amplification reaction means amounts of the ingredients such that the amplification reaction is not substantially restricted by the concentrations of the ingredients. A cofactor such as Mg²⁺, and dATP, dCTP, dGTP, and dTTP are required to be provided to the reaction mixture to such an extent that a desired degree of amplification can be achieved. All the enzymes used in the amplification reaction may be in an active state under the same reaction conditions. Indeed, a buffer allows all enzymes to be under conditions near optimal reaction conditions. Therefore, the amplification of the present invention may be performed in a single reaction material without changing conditions, such as the addition of a reaction material.

According to an embodiment of the present invention, the nucleic acid amplification reaction used in the present invention is a real-time nucleic acid amplification reaction.

The real-time nucleic acid amplification reaction may be performed using a non-specific fluorescence dye that non-specifically intercalates into a duplex, which is an amplicon of the target nucleic acid sequence.

In addition, the real-time nucleic acid amplification reaction may use a labeled probe specifically hybridizing with the target nucleic acid sequence. Examples of the method include a molecular beacon method using a dual-labeled probe forming a hair-pin structure (Tyagi et al, Nature Biotechnology v. 14 Mar. 1996), a hybridization probe method using two probes each single-labeled with a donor or an acceptor (Bernad et al, 147-148 Clin Chem 2000; 46), a Lux method using a single-labeled oligonucleotide (U.S. Patent No. 7,537,886), and a TaqMan method using a cleavage reaction of a double-labeled probe by 5'-nuclease activity of DNA polymerase as well as the hybridization of a dual-labeled probe (U.S. Patent No. 5,210,015 and No. 5,538,848), but are not limited thereto.

In addition, the real-time nucleic acid amplification reaction may be conducted using a duplex dependently formed on the presence of the target nucleic acid sequence. The duplex dependently formed on the presence of the target nucleic acid sequence is not an amplicon itself of the target sequence formed by the amplification reaction, and the amount of the duplex increases in proportion to the amplification of the target nucleic acid sequence. The duplex formed depending on the presence of the target nucleic acid sequence may be obtained by various methods, for example, PTO cleavage and extension (PTOCE) disclosed in WO 2012/096523, the contents of which are incorporated herein by reference.

According to an embodiment of the present invention, the nucleic acid amplification reaction composition comprises primers, a probe, and enzymes. The enzymes include, for example, Taq DNA polymerase and reverse transcriptase. The nucleic acid amplification reaction composition comprises a buffer, dNTP, KCI, and MgCl₂. For example, the nucleic acid reaction composition may comprise 40 mM Tris · CI(pH 8.8), 100 mM KCI, 4 mM MgCl₂, and 400 µM dNTP.

### Step (c): Determining presence or absence of respiratory pathogen

Finally, the presence or absence of the respiratory pathogen is determined by analyzing the results of the nucleic acid amplification reaction.

For example, when a signal above a particular threshold value is observed in the real-time detection of signals during the real-time nucleic acid amplification reaction, it is determined that a respiratory pathogen is present.

According to an embodiment of the present invention, the respiratory pathogen detected by the present invention is respiratory viruses and/or respiratory bacteria.

For example, the present invention determines an infection by influenza virus (*e.g.*, influenza A virus and influenza B virus), respiratory syncytial virus (RSV) (*e.g.,* RSV A and RSV B), adenovirus, enterovirus, parainfluenza virus (PIV) (*e.g.,* PIV 1, PIV 2, PIV 3, and PIV 4), metapneumovirus (MPV), bocavirus, rhinovirus, coronavirus (e.g., CoV NL63, CoV 229E, CoV OC43, CoV HKU1, SARS-CoV, MERS-CoV, SARS-CoV-2), Mycoplasma pneumoniae, Chlamydophila pneumoniae, Legionella pneumophila, Haemophilus influenzae, Streptococcus pneumoniae, Bordetella pertussis and/or Bordetella parapertussis.

According to an embodiment of the present invention, the present invention determines an infection by respiratory virus including influenza virus, respiratory syncytial virus (RSV), adenovirus, enterovirus, parainfluenza virus (PIV), metapneumovirus (MPV), bocavirus, rhinovirus, and/or coronavirus. More specifically, a kit of the present invention determines an infection by severe acute respiratory syndrome coronavirus 2 (SARS-CoV-2).

According to an embodiment of the present invention, the presence of SARS-CoV-2 in step (c) is determined when all of E gene, RdRP gene, and N gene of SARS-CoV-2 are measured to be positive.

Alternatively, the presence of SARS-CoV-2 in step (c) is determined when at least one of RdRP gene and N gene is measured to be positive.

According to an embodiment of the present invention, steps (b) and (c) are performed by a real-time nucleic acid amplification reaction showing a limit of detection (LoD) value of 100 RNA copies/reaction.

In another aspect of the present invention, there is provided a sample transport kit for detecting a respiratory pathogen, the kit including: (a) a nasopharyngeal swab; (b) a saliva swab and (c) a tube containing a sample transport medium.

Since the sample transport kit of the present invention is used for implementing the above-described method of the present invention, the common descriptions between them are omitted in order to avoid undue redundancy leading to the complexity of the present specification.

According to an embodiment of the present invention, a fibrous layer at the end of the nasopharyngeal swab has a thickness of 3-4.0 mm and a fibrous layer at the end of the saliva swab has a thickness of 2.5-5.5 mm.

According to an embodiment of the present invention, the fibrous layer disposed at the end of the nasopharyngeal swab includes a head part fibrous layer and a pillar part fibrous layer, and the head part fibrous layer is a fibrous layer with a thickness of 3.7-3.9 mm and the pillar part fibrous layer is a fibrous layer with a thickness of 3.0-3.1 mm.

According to an embodiment of the present invention, the fibrous layer of the saliva swab includes a horizontal long axis portion and a horizontal short axis portion, and wherein the thickness of the fibrous layer of the horizontal long axis portion is largest, the thickness of the fibrous layer of the horizontal short axis portion gradually decreases toward vertices of a long axis and the thickness of the fibrous layer of the horizontal short axis portion is smallest at the vertices of the long axis.

According to an embodiment of the present invention, the thickness of the fibrous layer of the saliva swab is 4.8-5.2 mm at the horizontal long axis portion and 2.8-3.2 mm at the horizontal short axis portion.

According to an embodiment of the present invention, the fibers of the fibrous layer have a Dtex value of 3.00-3.50. More specifically, the fibers of the fibrous layer have a Dtex value of 3.30-3.35.

According to an embodiment of the present invention, the sample transport medium comprises (i) a chaotropic agent, (ii) a detergent, (iii) a chelator, and (iv) a buffer.

Optionally, the sample transport medium further comprises a pH indicator (*e.g.*, phenol red, bromocresol, and bromothymol blue).

### Advantageous Effects of Invention

The features and advantages of the present invention are summarized as follows.
(a) Improved detection rates for positives and more accurate diagnostic results for respiratory pathogens can be obtained when a nasopharyngeal sample and a saliva sample, collected by swabbing, are dipped together into a sample transport medium and then nucleic acid molecules released from the swabs are used, compared with when the nasopharyngeal sample or the saliva sample is used alone.
(b) A saliva swab sample can be easily obtained in a non-invasive manner, and thus the use of such saliva swab sample together with a nasopharyngeal sample for respiratory pathogen detection, compared with the use of only the nasopharyngeal sample, can lead to improved detection rates of positives and more accurate detection results.

### Brief Description of Drawings

FIG. 1A illustrates a nasopharyngeal swab usable in the present invention.
FIG. 1B illustrates a saliva swab usable in the present invention.
FIG. 2A illustrates how to collect a sample by using the nasopharyngeal swab.
FIG. 2B illustrates how to collect a sample by using the saliva swab.

### Mode for Carrying out the Invention

The present invention will now be described in further detail by examples. It would be obvious to those skilled in the art that these examples are intended to be more concretely illustrative and the scope of the present invention as set forth in the appended claims is not limited to or by the examples.

### EXAMPLES

To investigate whether the detection rate of COVID-19 viruses was improved when two samples collected by a nasopharyngeal swab and a saliva swab were mixed and diagnosed, compared with when the two samples were separately subjected to coronavirus-19 detection, clinical trials were conducted under the Kyungpook National University IRB approval number 2020-03-041. Nasopharyngeal and saliva samples were collected from each of the patients who were confirmed with COVID-19 and hospitalized at Kyungpook National University Hospital, and subjected to COVID-19 virus identification by RT-PCR. As for each of 16 positive patients with a Ct value of 37 or less, 100 µl of respective residual samples were mixed in one tube (final volume: 200 µl). Thereafter, the detection or non-detection for COVID-19 viruses was again identified by RT-PCR as for a "mixed sample" in which the nasopharyngeal swab residual sample and the saliva swab residual sample were mixed.

RT-PCR was performed by the following method. The nucleic acid isolation was conducted using Seegene's STARMag 96X4 Universal Cartridge Kit (Cat. No. 744300.4. UC384, Seegene Inc.) and the automated nucleic acid extraction system Microlab NIMBUS (Cat. No. 65415-02, Hamilton). The nucleic acid isolation was conducted according to the manuals from the manufacturers of nucleic acid isolation reagents and the operation manual of the system. The nucleic acid isolation was conducted using the inactivated samples (nasopharyngeal swab and saliva swab samples, 300 µl; and the mixed sample, 200 µl) contained in sample transport media.

The detection or non-detection of SARS-CoV-2 was assayed using the isolated nucleic acid and Seegene's Allplex 2019-nCoV Assay. The Allplex 2019-nCoV Assay kit shows a limit of detection (LoD) value of 100 RNA copies/reaction.

The real-time PCR protocol adopted in the detection process is shown in Table 1.

**TABLE 1**

| Stage | Number of cycles | Temperature | Time |
|---|---|---|---|
| Reverse transcription | 1 | 50°C | 20 min |
| Activation | 1 | 95°C | 15 min |
| Denaturation | 45 | 94°C | 15 sec |
| Annealing and extension | | 58°C | 30 sec |

In Table 1, the detection of fluorescence signal was performed every cycle at 58°C.

Table 2 shows analytes and fluorescent substances used to detect the analytes in the Allplex 2019-nCoV Assay kit.

**TABLE 2**

| Fluorescent substance | Analyte |
|---|---|
| FAM | E gene |
| HEX | Internal control |
| Cal Red 610 | RdRP gene |
| Quasar 670 | N gene |

When the E gene, RdRP gene, and N gene on Table 2 were measured to be positive, it is determined that SARS-CoV-2 was present. A Ct value of above 37 was determined as negative (that is, the cut-off Ct value being 37).

**TABLE 3**

| Nasopharyngeal swab sample | Saliva swab sample | Mixed sample | | Number of patients |
|---|---|---|---|---|
| Positive | Positive | 4 | 0 | 4 |
| Positive | Negative | 7 | 0 | 7 |
| Negative | Positive | 5 | 0 | 5 |

In the test results on Table 3, all of the E gene, RdRP gene, and N gene were measured to be positive in the mixed samples of all the 16 patients, and thus it could be determined that SARS-CoV-2 was present.

As for four among all the 16 patients, all of the E gene, RdRP gene, and N gene were measured to be positive in both the nasopharyngeal swab and saliva swab samples, and thus it could be determined that SARS-CoV-2 was present.

However, all of the E gene, RdRP gene, and N gene were measured to be positive in only the nasopharyngeal swab samples for seven patients and in only the saliva swab samples for five patients. Especially, the detection results of SARS-CoV-2 in the saliva swab samples indicate that significant results can be also obtained from saliva swabs, in addition to nasopharyngeal swabs commonly used in the art. The above results show that the detection rate of SARS-CoV-2 can be improved when a mixed sample of a nasopharyngeal swab sample and a saliva swab sample is used.

Therefore, for the improvement in detection of SARS-CoV-2 and the enhancement in accuracy of testing, the present invention capable of sampling both nasopharyngeal swab samples and saliva swab samples is expected to be very advantageously used.

## Claims

1. A method for detecting a respiratory pathogen, the method comprising:
(a) dipping a nasopharyngeal swab and a saliva swab with collected samples into a sample transport medium contained in a tube;
(b) performing a nucleic acid amplification reaction using nucleic acid molecules obtained from the samples released from the nasopharyngeal swab and saliva swab and a nucleic acid amplification reaction composition; and
(c) analyzing the results of the nucleic acid amplification reaction to determine the presence or absence of the respiratory pathogen.

2. The method according to claim 1, wherein a fibrous layer at the end of the nasopharyngeal swab has a thickness of 3-4.0 mm and a fibrous layer at the end of the saliva swab has a thickness of 2.5-5.5 mm.

3. The method according to claim 2, wherein the fibrous layer at the end of the nasopharyngeal swab comprises a head part fibrous layer and a pillar part fibrous layer, and the head part fibrous layer is a fibrous layer with a thickness of 3.7-3.9 mm and the pillar part fibrous layer is a fibrous layer with a thickness of 3.0-3.1 mm.

4. The method according to claim 2, wherein the fibrous layer of the saliva swab comprises a horizontal long axis portion and a horizontal short axis portion, and wherein the thickness of the fibrous layer of the horizontal long axis portion is largest, the thickness of the fibrous layer of the horizontal short axis portion gradually decreases toward vertices of a long axis, and the thickness of the fibrous layer of the horizontal short axis portion is smallest at the vertices of the long axis.

5. The method according to claim 4, wherein the thickness of the fibrous layer of the saliva swab is 4.8-5.2 mm at the horizontal long axis portion and 2.8-3.2 mm at the horizontal short axis portion.

6. The method according to claim 2, wherein fibers of the fibrous layer have a Dtex value of 3.00-3.50.

7. The method according to claim 6, wherein the fibers of the fibrous layer have a Dtex value of 3.30-3.35.

8. The method according to claim 1, wherein the sample transport medium comprises (i) a chaotropic agent, (ii) a detergent, (iii) a chelator, and (iv) a buffer.

9. The method according to claim 8, wherein the sample transport medium further comprises a pH indicator.

10. The method according to claim 1, wherein the nucleic acid amplification reaction is a real-time nucleic acid amplification reaction.

11. The method according to claim 1, wherein the nucleic acid amplification reaction composition comprises primers, a probe, and enzymes.

12. The method according to claim 1, wherein the respiratory pathogen is respiratory virus and/or respiratory bacteria.

13. The method according to claim 12, wherein the respiratory virus is influenza virus, respiratory syncytial virus (RSV), adenovirus, enterovirus, parainfluenza virus (PIV), metapneumovirus (MPV), bocavirus, rhinovirus and/or coronavirus.

14. The method according to claim 13, wherein the coronavirus is severe acute respiratory syndrome coronavirus 2 (SARS-CoV-2).

15. The method according to claim 14, wherein the presence of SARS-CoV-2 in step (c) is determined when all of E gene, RdRP gene, and N gene of SARS-CoV-2 are measured to be positive.

16. The method according to claim 14, wherein the presence of SARS-CoV-2 in step (c) is determined when at least one of RdRP gene and N gene in the RdRP gene and N gene is measured to be positive.

17. The method according to claim 1, wherein steps (b) and (c) are performed by a real-time nucleic acid amplification reaction showing a limit of detection (LoD) value of 100 RNA copies/reaction.

18. A sample transport kit for detecting a respiratory pathogen, the kit comprising: (a) a nasopharyngeal swab; (b) a saliva swab; and (c) a tube comprising a sample transport medium.

19. The kit according to claim 18, wherein a fibrous layer at the end of the nasopharyngeal swab has a thickness of 3-4.0 mm and a fibrous layer at the end of the saliva swab has a thickness of 2.5-5.5 mm.

20. The kit according to claim 19, wherein the fibrous layer at the end of the nasopharyngeal swab comprises a head part fibrous layer and a pillar part fibrous layer, and the head part fibrous layer is a fibrous layer with a thickness of 3.7-3.9 mm and the pillar part fibrous layer is a fibrous layer with a thickness of 3.0-3.1 mm.

21. The kit according to claim 19, wherein the fibrous layer of the saliva swab comprises a horizontal long axis portion and a horizontal short axis portion, and wherein the thickness of the fibrous layer of the horizontal long axis portion is largest, the thickness of the fibrous layer of the horizontal short axis portion gradually decreases toward vertices of a long axis and the thickness of the fibrous layer of the horizontal short axis portion is smallest at the vertices of the long axis.

22. The kit according to claim 21, wherein the thickness of the fibrous layer of the saliva swab is 4.8-5.2 mm at the horizontal long axis portion and 2.8-3.2 mm at the horizontal short axis portion.

23. The kit according to claim 19, wherein fibers of the fibrous layer have a Dtex value of 3.00-3.50.

24. The kit according to claim 19, wherein fibers of the fibrous layer have a Dtex value of 3.30-3.35.

25. The kit according to claim 18, wherein the sample transport medium comprises (i) a chaotropic agent, (ii) a detergent, (iii) a chelator, and (iv) a buffer.

26. The kit according to claim 17, wherein the sample transport medium further comprises a pH indicator.
